# EUROPEAN PATENT SPECIFICATION

(11) **EP 4 416 567 B1**
(45) Date of publication and mention of the grant of the patent: **12.03.2025**
(21) Application number: 22801810.7
(22) Date of filing: 14.10.2022
(51) Int. Cl.: G05D 11/00, B01F 23/40

(54) **METHOD FOR MONITORING A LIQUID CULTURE FLOW**
VORRICHTUNG ZUM INLINE-MISCHEN VON FLÜSSIGKEITSSTRÖMEN
DISPOSITIF DE MÉLANGE EN LIGNE DE FLUX LIQUIDES

(30) Priority: 14.10.2021 EP 21202676
(43) Date of publication of application: 21.08.2024
(73) Proprietor: Ovizio Imaging Systems NV/SA, 1310 La Hulpe (BE)
(72) Inventor: LEBACQ, André, 1030 Brussels (BE)
(74) Representative: De Clercq & Partners
(86) International application number: PCT/EP2022/078658
(87) International publication number: WO 2023/062193

(56) References cited:
- US-A- 5 630 706
- US-A1- 2003 052 007
- US-A1- 2004 049 301
- US-B1- 6 498 862

## Description

### Field of the invention

The present invention is in the field on inline (continuous flow) mixing, in particular for applications in need of accurate quantitative inline mixing or dilution. In particular for applications in need of accurate quantitative inline dilution of a liquid culture for monitoring of the liquid culture.

### Background to the invention

Devices for inline mixing of two or more liquid flows provide a continuous stream of mixed liquids. For example, an inline static mixer provides a way to output an homogeneous mix of the two or more liquid inputs flow. The liquid flow in each stream is controlled by separate pumps. Pumps do not have a constant discharge flow rate. They exhibit a pulsating flow rate caused by a flow inducer, such as a peristaltic pump roller shoe, or piston pump valve, or by other mechanical interference. This is reflected in a pulsating output pressure. A problem caused by periodic flow rate variation is inaccurate inline mixing. Quantitative inline mixing is desirable in many applications. It involves calculating the volume of liquid being mixed from each flow channel, so that the outlet flow has a known volumetric composition. Having a known volumetric composition is critical in many applications. For instance, in continuous optical monitoring the sample is diluted inline and optical concentration measurements of the mixed liquids are continuously recorded; the concentration of the original undiluted sample is calculated from knowledge of the dilution. The inventors have found that the presence of a mixer introduces a significant error into the mixing calculation that cannot be correct from a knowledge of separate flow rates. The problem solved by the present invention is how to improve the accuracy of dilution of an inline mixer.

### Summary of the invention

The invention is defined by the appended claims.

### Figure Legends

**FIG. 1** is a schematic illustration of the device provided herein; the inlet flow channels merge into a single channel.
**FIG. 2** is similar to FIG. 1 with the exception that both inlet flow channels connect directly to the mixer.
**FIG. 3** is similar to FIG. 1 wherein a flow rate sensor is provided in each inlet flow channel.
**FIG. 4** is similar to FIG. 1 wherein pulsation dampener comprises a gas-expansion chamber.
**FIG. 4A** is a schematic detail of the gas-expansion chamber.
**FIG. 5****,** is similar to FIG. 1 wherein pulsation dampener comprises an inline complaint tube.
**FIG. 5A** is a schematic detail of the inline complaint tube.
**FIG. 6** is a schematic illustration of the device provided herein having an array of selectable pulsation dampeners (132ac, 134ac).
**FIG. 7** is similar to FIG. 1 wherein each of the inlet flow channels is provided with a bubble trapper.
**FIG. 7A** is a schematic detail of the bubble trapper.
**FIG. 8** shows an example of the device with three inlet flow channels.

### Detailed description of invention

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

The term "upstream" has used herein refers to a direction that is towards a pump (and away from a final outlet). The term "downstream" has used herein refers to a direction that is away from the pump (and towards the final outlet).

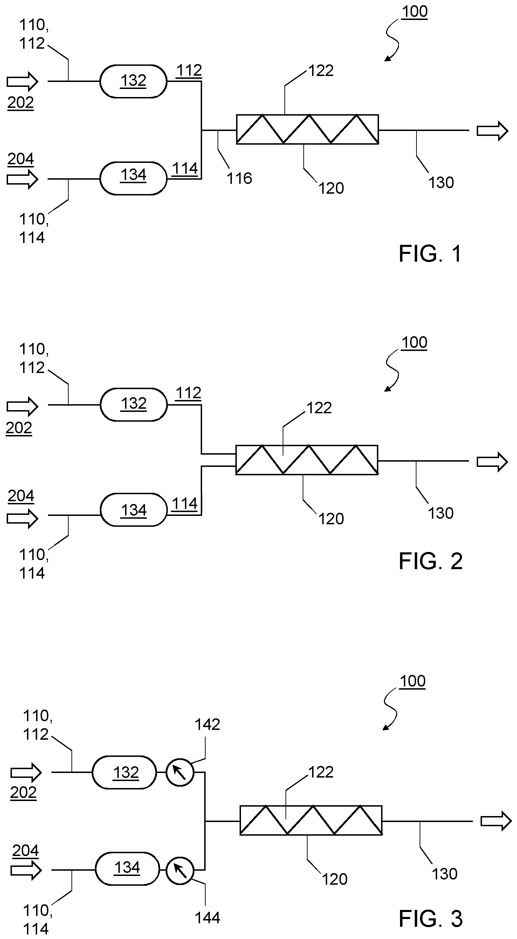

Provided herein is a device (100) for inline mixing of two or more liquid flows, wherein each liquid flow is induced by a separate pump (202, 204). The basic device is exemplified in **FIGs. 1** to **6****.** The device (100) is preferably used in a method for monitoring a liquid culture, wherein the monitoring comprises inline dilution of a flow of the liquid culture using the device, wherein one of the liquid flows is the liquid culture and one of the liquid flows is a diluent.

The device (100) comprises a set (110) of inlet flow channels (112, 114) each configured for conducting a separate liquid flow. Each inlet flow channel (112, 114) has an inlet (IFC inlet) and an outlet (IFC outlet). The liquid flow enters each inlet flow channel (112, 114) from a separate pump (202, 204).

The device (100) further comprises a mixer (120) into which liquid from the set (110) of inlet flow channels (112, 114) enters and is mixed.

The mixing ratio, and the fraction of each inlet material in the mixed output, can be accurately controlled by controlling the pumping speed in each inlet. Increasing the pumping speed in one of the inlet channels (while keeping the pumping speed in the other inlets unchanged) will increase the volumetric and mass fraction of the liquid fed into that inlet in the mix output from the device (100). On the other hand, decreasing the pumping speed in one of the inlet channel will decrease the volumetric and mass fraction of the material fed into that inlet in the mix output from the device (100).

The device (100) may further comprise an outlet flow channel (130) configured for conducting the mixed liquid flow from the mixer (130). The outflow channel (130) may connect to a further downstream processing step such as measurement (*e.g.* optical, spectroscopic, conductance, biochemical or chemical assay), liquid handling (*e.g.* dispensing robot), liquid packaging (*e.g.* vial filler).

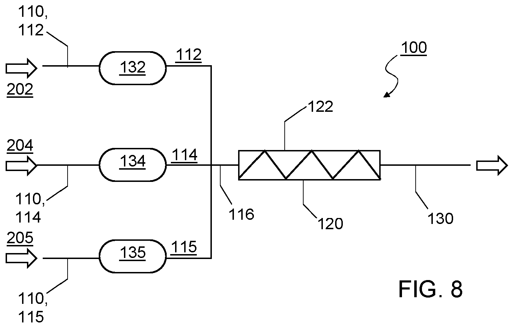

The passage of liquid flow through each inlet flow channel (112, 114) effects a reduction in periodic variability (pulsations) in liquid flow rate. Each inlet flow channel (112, 114) of the set (110) may be disposed with a pulsation dampener (132, 134), configured to effect the reduction in periodic variability in liquid flow rate from each inlet flow channel (112, 114) of the set (110). The number of inlet flow channels (112, 114) in the set (110) may be 2 or more, for instance, 2, 3, 4, 5 or more. **FIGs. 1** to **4****,** and **5** to **7** show examples having two inlet flow channels (112, 114); **FIG. 8** shows an example with three inlet flow channels (212, 214, 215), each containing a pulsation dampener (132, 134, 135).

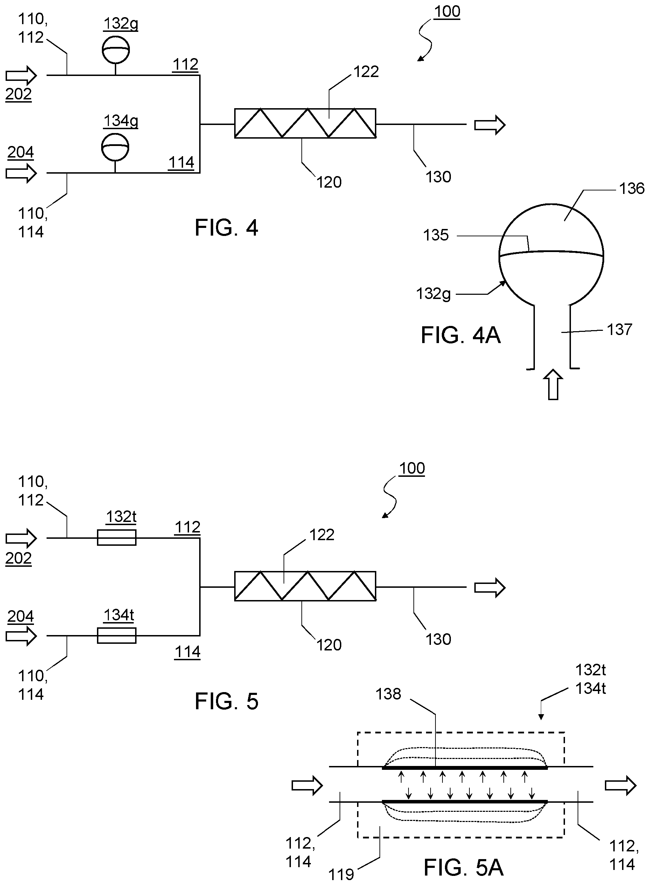

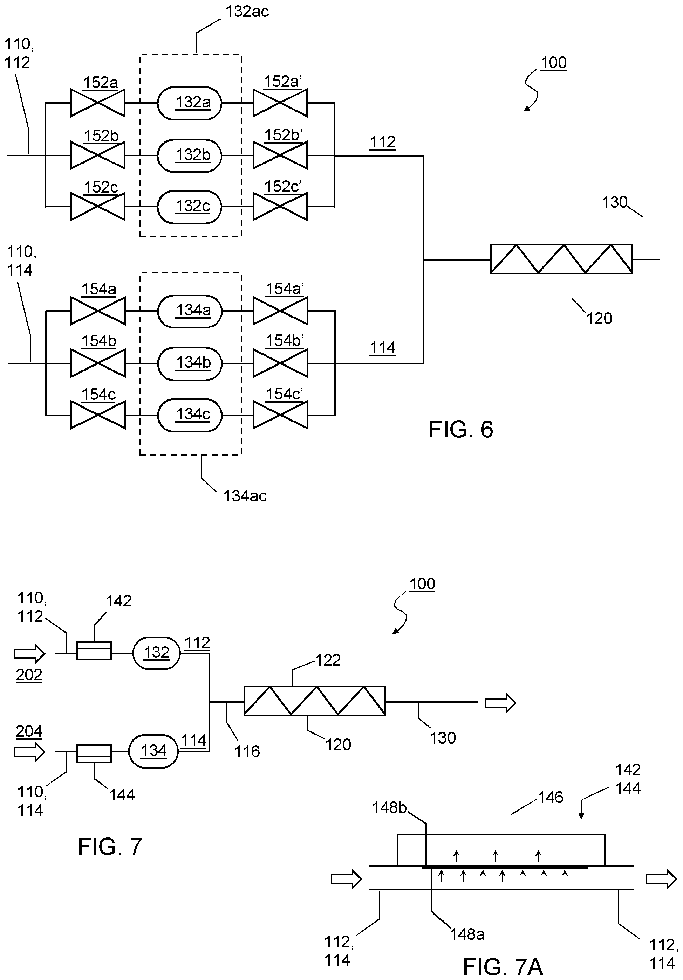

**FIGs. 1** to **6** each show a possible configuration of the device (100) having a set (110) of two inlet flow channels (112, 114) each with a pulsation dampener (132, 134) that lead into a mixer (120), and an outlet flow channel (130) carrying the mixed liquids. In **FIG. 1****,** the inlet flow channels (112, 114) merge into a single channel (116) that connects to the mixer (120). In **FIG. 2** the inlet flow channels (112, 114) both connect independently and directly to the mixer (120). In **FIG. 3** a flow rate sensor (142, 144) is provided in each inlet flow channel (112, 114) downstream of the pulsation dampener (132, 134) and upstream of the mixer (130). In **FIG. 4****,** the pulsation dampener (132, 134) comprises a gas-expansion chamber. In **FIG. 5****,** the pulsation dampener (132, 134) comprises an inline complaint tube. In **FIG. 6****,** the device is provide with an array of selectable pulsation dampeners (132ac, 134ac).

The device (100) described herein is for inline mixing of two or more liquid flows. By inline mixing it is meant that for a flow period the two or more liquid flows continuously enter the device, and a mixed liquid continuously exits the device. It may also be referred to as a continuous stream mixer, or inline mixer. It is understood for many applications, the flow period is punctuated by a pause period during which flow into the device is paused.

The pump (202, 204) is any that consumes energy (*e.g.* electrical energy, pneumatic pressure, hydraulic pressure), to induce a liquid flow. Types of pump for an inline mixing application include a peristaltic pump and piston pump. One pump is provided per inlet flow channel (112, 114). The device (100) may further comprise the respective pumps (202, 204).

Energy-consuming pumps do not have a constant discharge flow rate. They are typically known as a pulsations pump. They exhibit a pulsating flow rate caused by a flow inducer, such as a peristaltic pump roller shoe, or piston pump valve, or by other mechanical interference. There is a periodic high-low/no flow rate cycle. This is reflected in a pulsating output pressure. The problem when the flow rates in inlets are pulsating is that they are not steady state. The mixer introduces an unknown effect, and especially an unknown delay, between the flows measured in inlet flow channels (110) and the partial flows constituting the mixed output (130) which makes it difficult to compute the actual mixing ratio of each constituent flow.

The output of the pumps could be sent to an intermediate mixed tank, and the mix withdrawn from the tank for measurement or other purposes, but this setup would create issues such as difficulty of emptying the tank (potential cross contamination of samples with different mixing ratio), difficulty of cleaning and potential clogging, increased cost, etc.

Quantitative inline mixing is desirable in many applications. It involves calculating the volume of liquid being mixed from each flow channel, so that the outlet flow has a known volumetric composition. In other words the volume of each inlet liquid is known in the outputted flow stream. Having a known volumetric composition is critical in many applications. For instance, in continuous (*e.g.* optical) monitoring the sample is diluted inline and (*e.g.* optical) concentration measurements of the mixed liquids are continuously recorded; the concentration of the original undiluted sample is calculated from knowledge of the dilution.

The inventors have found that the presence of a mixer introduces an unknown delay, that can be different for each inlet between the flow produced by the pump (and possibly measured by the flow sensors), and the fraction of each inlet flow constituting the mixed output. Factors influencing the delay are numerous and include flow properties of the inlet material and the pump pulsating frequency. Even if the flow rates in each inlet flow channel (112, 114) are measured with inline flow rate sensors (142, 144), a correction applied to account for the flow rate variation would yield an erroneous measurement of the mixing ratio. The mixer may also introduce an unknown averaging effect further increasing the difficulty of accurately measuring the output mixing ratio.

The inventors have solved the problem of accurate inline mixing by disposing each inlet flow channel (112, 114) of the set (110) with a pulsation dampener (132, 134) configured to reduce variability or pulsations in liquid flow rate from liquid exiting the inlet flow channel (112, 114). Dampening the flow pulsations leads to more constant flow rate for a duration of the passage through the mixer. The flows presented at the mixer input are steady state, allowing mixing ratio to be accurately computed based on the steady flow rates values.

The pulsation dampener (132, 134) is a device that reduces or eliminate periodic variations in liquid flow rate. Liquid flow entering the inlet flow channel (112, 114) has a pulsating flow rate, and the flow rate of liquid exiting the inlet flow channel (112, 114) is more constant over time. Pulsation dampener (132, 134) has a pulsation dampener inlet (PD inlet), for entry of the liquid flow from the inlet flow channel (112, 114). The pulsation dampener (132, 134) may have an pulsation dampener outlet (PD outlet), liquid flow leaves the pulsation dampener (132, 134) and returns to the inlet flow channel (112, 114). Suitable pulsation dampeners include those including those known in the art. The skilled person will understand how one should be optimally configured for a particular flow rate and pressure.

The pulsation dampener (132, 134) may comprise a gas-filled expansion chamber ("gas-based"), wherein the gas expands or contracts responsive to hydraulic force transmitted by the liquid flow in the inlet flow channel (112, 114). It acts as a gas cushion. A possible configuration is shown in **FIG. 4** with gas-based pulsation dampeners indicated (132g, 134g) and a detail of a pulsation dampener (132g) is shown in **FIG. 4A****.** A diaphragm (135) may separate the expansion chamber (136) from the inlet flow channel (112, 114). There may be no diaphragm separating the expansion chamber from the inlet flow channel (112, 114). A conduit (137) may connect the gas-filled expansion chamber with the inlet flow channel (112, 114). The gas-filled expansion chamber may initially be placed under negative pressure to prime it.

The pulsation dampener (132, 134) may comprises an inline compliant (elastic-walled) tube wherein the tube expands or contracts responsive to changes in hydraulic force transmitted by the liquid flow in the inlet flow channel (110, 112, 114). The liquid flow may pass inside a lumen of the compliant (elastic-walled) tube. It passes out from the pulsation dampener (132, 134) back into the inlet flow channel (112, 114). A possible configuration is shown in **FIG. 5** with tube-based pulsation dampeners indicated (132t, 134t) and a detail of a pulsation dampener (132t) is shown in **FIG. 5A****.** A compliant tube (138) is connected inline to inlet flow channel (112) that expands (see arrows) or contracts responsive to pressure in the inlet flow channel (112). A chamber (119) may or may not surround the compliant tube (134). An example of a chamber-less tube-based pulsation dampener is one manufactured by Sensirion (Sensirion Liquid Flow Pulsation Damping Kit).

The skilled person will understand how to optimally configure a single pulsation dampener. As understood in the art, a pulsation dampener acts as a low pass filter on the pump pulsation. If and when the pump speed changes, the flow at the pulsation dampener output will typically follow the pump speed change with a delay. This delay will be larger when the dampener is optimised for lower flow rates. There is a trade-off between dampening effectiveness and response time; the response time of the pulsation dampener is typically optimised to be fast within certain a certain flow rate range. A pulsation dampener optimised for a small flow rate will also function at larger flow rates, however, the response time is less optimised. When the pump speed is changed abruptly, the pressure inside the dampener and the flow at the dampener outlet must adapt to the new pressure and flow conditions at the dampener inlet. For example, when decreasing the pump speed, the liquid held in the dampener must discharge through the outlet circuit which will take time, depending on the fluidic resistance of the outlet circuit. Stepping from a low diluent flow rate to a larger diluent flow rate is also subject to the dampener response time: the outlet flow rate will stabilize to the new higher flow rate following the pump speed change only after a delay.

A situation arises for instance when monitoring continuously growth of a cell culture; as cell density increases gradually over time, more diluent is needed to dilute the cell culture within a measurement range of a downstream instrument (*e.g.* DHM or spectrophotometry). Another situation is when the mixing device input is multiplexed to successively connect to multiple cell cultures with various concentration to monitor. In this case, when switching from one culture to another, the dilution ratio may need to be different, but the response time for switching from one diluent flow rate to another may be unacceptably long for the application. Another situation when multiple selectable pulsation dampeners may be needed is for example when the range of flow rates needed to address an application is larger than the range of flow rates that a single dampener can dampen with acceptable remaining pulsation in the outlet flow and response time. In this case, the pulsation dampeners have different characteristics to address different ranges of flow rates.

The device (100) may be provided with a set (132ac or 134ac) of selectable pulsation dampeners (132a-c; 134a-c) one or more of which can be brought into use without manually swapping-out the existing pulsation dampener. Each pulsation dampener has an internal pressure state corresponding to one particular pumping speed (and outlet flow rate). When the pump speed is changed abruptly, swapping to the corresponding pulsation dampener allows a drastically reduced system response time for the pressure inside the dampener to adapt to the new pumping speed.

As shown, for instance, in **FIG. 6****,** an inlet flow channel (112, 114) may be provided with a set (132ac or 134ac) of at least two (e.g. 2, 3, **4,** 5 or more) selectable pulsation dampeners (132a-c; 134a-c). Each pulsation dampener (132a-c; 134a-c) in the set (132ac, 134ac) may have a different performance or internal pressure state. By selectable, it is meant that one or more of the pulsation dampeners *(e.g.* 132a; 134a) can be temporarily activated *(e.g.* by one or more valves) to dampen the flow variation, while others (e.g. 132b-c; 134b-c) in the set can be temporarily de-activated and/or isolated (*e.g.* by a valve) (and hence have no dampening effect). Selectability allows activation of a pulsation dampener (132a-c; 134a-c) according to one or parameters of the liquid flow in order to optimise the system response time or flow rates range. Each pulsation dampener (132a-c; 134a-c) in the set may have a different performance, for instance, a capacity to work at different flow rates or viscosities or reaction times. In additional, each pulsation dampener may keep a pressure state corresponding to a particular pumping speed (by the way of two valves to completely isolate the dampener when not in use). Higher flow rates give rise to larger hydraulic forces, and can be accommodated by using larger expansion chamber or less compliant materials.

The set of selectable pulsation dampeners (132a-c; 134a-c) may be connected in parallel. The set of selectable pulsation dampeners (132a-c; 134a-c) may form a parallel array having an inlet (PA inlet) and outlet (PA outlet). The IFC inlet connects with the PA inlet and the PA outlet connects with the IFC inlet.

Each pulsation dampener (132a-c; 134a-c) in the set may be disposed at least one flow control valve (152a-c; 154a-c) (*e.g.* on/off valve, variable flow rate valve) to control activation and de-activation of the pulsation dampener. The flow control valve (152a-c; 154a-c) may be any, including one known in the art. Examples include a pinch valve, for instance manufactured by Clippard.

Each pulsation dampener (132a-c; 134a-c) in the set may be disposed at an inlet end with a flow control valve (152a-c; 154a-c) to control activation and de-activation of the pulsation dampener.

Where the pulsation dampener (132a-c; 134a-c) comprises the gas-filled expansion chamber having a single inlet, one flow control valve may be provided to control flow of liquid into the single inlet.

Where the pulsation dampener (132a-c; 134a-c) is of the tubular type having both an inlet and outlet, each pulsation dampener (132a-c; 134a-c) in the set may be flanked by a flow control valve. In other words, one flow control valve may be provided to control flow of liquid into the pulsation dampener inlet, and one flow control valve may be provided to control flow of liquid from the pulsation dampener outlet.

A controller may be provided, configured for automatic selection of a selectable pulsation dampeners (132a to 132c, 134a to 134c) responsive to flow rate through the inlet flow channels (112, 114). The controller may implement a closed feedback loop.

The device (100) may be disposed with one or more bubble trappers (142, 144) configured to remove air bubbles from a liquid flow. An exemplary configuration is shown in **FIGs. 7** and **7A****.** The one or more bubble trappers may be placed before or after the pump (202, 204) or both before and after. Those placed after the pump may be placed in an inlet channel (112, 114). Those placed after the pump may be placed in an inlet channel (112, 114) and before the pulsation dampener (132, 134). At least one, preferably each and every liquid flow may be provided with one or more bubble trappers placed before the pump (202, 204). At least one, preferably each and every liquid flow may be provided with one or more bubble trappers placed after the pump (202, 204); this is shown in the exemplary configuration of **FIG. 7****.** At least one, preferably each and every inlet channel (112, 114) may be provided with one or more bubble trappers.

A bubble trapper (142, 144) may comprise a permeable membrane (146) having liquid-flow contact side (148a) and an opposing gas-exhaust side (148b). The detail is shown in **FIG. 7A****.** Liquid passes through an inlet on the liquid-flow contact side (148a) and contact and flows over (parallel to) the permeable membrane (146) and out through an outlet on the liquid-flow contact side (148a). The back pressure created by the pump creates an over pressure on the liquid-flow contact side (148a) of the membrane (146) with respect to the gas exhaust side of the membrane (146). The air contained in the pumped liquid is pushed through the permeable membrane (146) to the exhaust side and the liquid passes through the outlet cleared of any air bubbles.

A bubble trapper (142, 144) may be in an passive or active configuration. In a passive configuration, the exhaust side is held at atmospheric pressure. In an active configuration, the exhaust side is at negative pressure (under vacuum).

The mixer (120) is configured to mix the two or more liquid flows. The mixer (120) is an inline mixer. The mixer comprises one or more mixer inlets that feed into a mixing chamber (122), and a mixer outlet connected to the outlet flow channel (130). The mixing chamber may be longitudinal. A plurality of mixers may be provided; they may be connected in series or parallel.

The mixer (120) produces continuously a flow of mixed liquids to the outlet flow channel (130). The mixer may be passive (static) - does not contain a powered mixing element. The mixer may be active - contains a powered mixing element (*e.g.* magnetic bar, stirrer blade). The mixer may be configured to produce a turbulent flow of the entering one or more liquid flows to enhance mixing efficiency. The mixer may be configured to direct the flow of the two or more liquid flows into the chamber such that they are towards (against) each other. The mixer may be configured to direct the flow of the two or more liquid flows into the chamber such that they are in the same longitudinal direction. The mixer may contain a helical flow guide - a longitudinal array of helical segments each of one turn or less, and each adjacent helical segment being mutually axially rotationally offset. A helical flow guide is found typically in helical static mixer, and is known in the art. The mixer (120) may be any, including one known in the art. Examples of suitable mixers (120) include those manufactured by Koflo.

The inlet flow channels (112, 114) may merge into a single channel (116) that connects to the mixer (120) as shown for instance in **FIGs. 1**, **3 to 6**. The inlet flow channels (112, 114) may both connect directly to the mixer (120) as shown for instance in **FIG. 2****.** Either configuration may be used in the various configuration described herein.

Each inlet flow channel (112, 114) may be provided with a flow rate sensor (142, 144) configured to measure the flow of liquid in the inlet flow channel (112, 114). See for instance, **FIG. 3**. Each flow rate sensor (142, 144) is located downstream of the pulsation dampener (132, 134). Each flow rate sensor (142, 144) is located upstream of the mixer (120). The flow rate sensor (142, 144) may be used to calculate the volumes of liquid flow being mixed. The flow rate sensors is preferably inline. The flow rate sensor (142, 144) may be any, including one known in the art. The skilled person in the art understands that a flow rate sensor is matched for measurement of fluid with compatible properties (density, viscosity, etc). Examples of suitable flow rate sensors include those manufactured by Bronkhorst.

Each inlet flow channel (112, 114) may be provided with a pressure sensor configured to measure the pressure liquid in the inlet flow channel (112, 114). Each pressure sensor is located downstream of the pulsation dampener (132, 134). Each pressure rate sensor is located upstream of the mixer (120). Where the flow rate sensor (142, 144) is also present, each pressure sensor is located upstream of the flow rate sensor (142, 144). The pressure sensor may be used to monitor the dampening effect of the pulsation dampeners (132, 134). The pressure sensor is preferably inline. The pressure sensor may be any, including one known in the art. Examples of suitable pressure sensors include those manufactured by Pendotech.

The device (100) may further comprise an inline optical measurement cell (350) (flow cell) for inline measurement of one or more optically-detectable properties of the diluted sample. The inline optical measurement cell is connected to the outlet flow channel (130).

Further provided is a digital holographic microscopy (DHM) system comprising:
- a DHM (300);
- the device (100) as described herein.

The DHM is adapted for (continuous) measurement of a sample in the inline optical measurement cell (350).

Digital Holographic Microscopy (DHM) is a technique which allows a recording of three dimensional information of a sample or object without the need of scanning the sample layer-by-layer. In this respect DHM is a superior technique to confocal microscopy in terms of acquisition speed. In DHM, a holographic representation is recorded by an image sensor such a CCD or CMOS. The holographic representation may be subsequently be stored or processed on a computer.

To make a holographic representation, or hologram, traditionally a light source that is coherent is used to illuminate the sample. The light source may be provided by a laser. In the most basic set-up, the light form the source is split into two beams, an object beam and a reference beam. The object beam is sent via an optical system to the sample and interacts with it, thereby altering the phase and amplitude of the light depending on the object's optical properties and 3D shape. The object beam which has been reflected on or transmitted through the sample, is then made (*e.g.* by set of mirrors and/or beam splitters) to interfere with the reference beam, resulting in an interference pattern which is digitally recorded. Since the hologram is more accurate when object beam and reference beam have comparable amplitude, an absorptive element can be introduced in the reference beam which decreases its amplitude to the level of the object beam, but does not alter the phase of the reference beam or at most changes the phase globally, i.e. not dependent on where and how the reference beam passes through the absorptive element. The recorded interference pattern contains information on the phase and amplitude changes which depend on the object's optical properties and 3D shape.

An alternative way of making a hologram is by using the in-line holographic technique. In-line DHM is similar to the more traditional DHM, but does not split the beam, at least not by a beam splitter or other external optical element. In-line DHM is most preferably used to look at a not-too-dense solution of particles, e.g. cells, in a fluid. Thereby some part of the at least partially coherent light will pass through the sample without interacting with the particles (reference beam) and interfere with light that has interacted with the particles (object beam), giving rise to an interference pattern which is recorded digitally and processed. In-line DHM is used in transmission mode, it needs light with a relatively large coherence length, and cannot be used if the samples are too thick or dense.

Another DHM technique called differential DHM (DDHM) is disclosed in European patent EP 1 631 788. DDHM is different to the other techniques in that it does not make use of reference and object beams in the traditional sense. In a preferred set-up of DDHM, the sample is illuminated by a light source that outputs at least partially coherent light for use in a reflection or in a transmission mode. The reflected or transmitted sample beam can be sent through an objective lens and subsequently split in two by a beam splitter and sent along different paths in a differential interferometer, *e.g.* of the Michelson or Mach-Zehnder type. In one of the paths, a beam-bending element or tilting mechanism is inserted, *e.g.* a transparent wedge or a diffraction grating. The two beams are then made to interfere with each other in the focal plane of a focusing lens and the interference pattern in this focal plane is recorded digitally by an image sensor such a CCD or CMOS. The interference pattern may be stored on a digital storage medium. Hereby, due to the beam-bending element, the two beams are slightly shifted in a controlled way and the interference pattern depends on the amount of shifting. Then the beam-bending element is turned, thereby altering the amount of shifting. The new interference pattern is also recorded. This can be done a number N of times, and from these N interference patterns, the gradient (or spatial derivative) of the phase in the focal plane of the focusing lens can be approximately computed. This is called the phase-stepping method, but other methods of obtaining the phase gradient are also known, such as a Fourier transform data processing technique. The gradient of the phase can be integrated to give the phase as a function of position. The amplitude of the light as a function of position can be computed from the possibly but not necessarily weighted average of the amplitudes of the N recorded interference patterns.

Since phase and amplitude are thus known, the same information is obtained as in a direct holographic method (using a reference and an object beam), and a subsequent 3D reconstruction of the object can be performed.

Another DHM technique uses an off-axis interferometer, as described, for instance, in WO2011042442A1. The off-axis interferometer comprises a recording plane (10), and a grating (G) located in a plane optically conjugated with said recording plane (10), said grating (G) defining a first and a second optical path, said optical path corresponding to different diffraction order.

A fringe contrast between two non-parallel temporally partially coherent light beams may be obtained by an incident light beam, said incident light beam being temporally partially coherent; focusing said incident light beam on the grating (G) for producing at least two diffracted light beams, focusing said diffracted light beams to the infinite for obtaining parallel diffracted light beams parallel to the incident light beam, focusing said parallel diffracted light beams on said recording plane, producing fringe contrast independent of the position in the recording plane.

Off-axis digital holograms may be obtained by
- providing a partially coherent light source producing a first partially coherent light beam; splitting the first partially coherent light beam into a second light beam and a third light beam; focusing the third light beam on the grating (G) for splitting said third light beam into a non-zero order diffracted light beam and a zero order diffracted light beam;
- focusing the non-zero order diffracted light beam and zero order diffracted light beam to the infinite in order to obtain parallel and spatially separated non- zero diffracted light beam and zero order diffracted light beam;
- stopping the zero order diffracted light beam;
- combining the non-zero diffracted light beam with the second light beam into a recombined beam;
- focusing the recombined beam on recording means to obtain an off-axis interferogram.

The light source may emit spatially and temporally partially coherent light. The light source may emit highly correlated laser light. Spatially and temporally partially coherent light may be produced by for instance a light-emitting diode (LED). A LED is cheaper than a laser and produces light with a spectrum centred around a known wavelength, which is spatially and temporally partially coherent, *i.e.* not as coherent as laser light, but still coherent enough to produce holographic images of the quality which is necessary for the applications at hand. LEDs also have the advantage of being available for many different wavelengths and are very small in size and easy to use or replace if necessary. Therefore, providing a method and system which can use spatially and temporally partially coherent light for obtaining holographic images will lead to more cost-effective devices for implementing such a method.

The use of DHM in a diagnostic setting has many advantages which makes it the ideal technique to implement in a setting such as in the current invention. Besides a bright field image, a phase shift image is also created. The phase shift image is unique for DHM and gives quantifiable information about optical distance. In reflection DHM, the phase shift image forms a topography image of the object.

An object image is calculated at a given focal distance. However, as the recorded hologram contains all the necessary object wave front information, it is possible to calculate the object at any focal plane by changing the focal distance parameter in the reconstruction algorithm. In fact, the hologram contains all the information needed to calculate a complete image stack. In a DHM system, where the object wave front is recorded from multiple angles, it is possible to fully characterize the optical characteristics of the object and create tomography images of the object.

The needed components for a DHM system are inexpensive optics and semiconductor components, such as a laser diode and an image sensor. The low component cost in combination with the auto focusing capabilities of DHM, make it possible to manufacture DHM systems for a very low cost. Nevertheless, the cost of a DHM may still be too high for monitoring a large amount of reactors. For this, the present invention provides a system comprising one DHM and a set of electro-fluidic circuits which are capable of guiding fluid samples from multiple reactors to the DHM and preferably back. Hereby, only one DHM is needed to monitor multiple reactors and the overall cost can be reduced.

Generally, a DHM comprises a light source that emits coherent light or at least partially coherent light such as a LASER or LED, an interferometer which may comprise a set of mirrors and/or beam splitters, and an image sensor such as a CCD or CMOS, and a processor and a computer-readable storage medium (*e.g.* solid state drive, flash card, or magnetic recording device). A DHM may also comprise further optical components such as one or more lenses, mirrors, prisms, attenuators, etc. A DHM may comprise or may be connected to processing means such as a mainframe, a PC, a logical device such as a PLC, etc. A DHM may work in transmission and/or reflection mode, depending on the nature of the sample which is to be observed. A DHM may be a traditional DHM, an in-line DHM, a differential DHM, or another kind of DHM.

The different flow channels (112, 114, 130) of the device may be provided as tubing connecting the pulsation dampers (132, 134) and mixer (120), and other components (*e.g.* valves, flow rate sensor, pressure sensor) according to a working configuration of the device (100). The tubing may be rigid, semi-rigid or flexible. The tubing may or may not be radially expandable.

The different flow channels (112, 114, 130) of the device may be provided in a monoblock connecting the pulsation dampers (132, 134) and mixer (120), and other components (*e.g.* valves, flow rate sensor, pressure sensor, bubble trapper) according to a working configuration of the device (100). The monoblock may be formed by additive manufacturing, moulding, milling and the like. The monoblock may be made from any suitable material (*e.g.* polymer, polycarbonate) Other components may also be formed in the monoblock such as the pulsation damper (132, 134) and mixer (120).

The different flow channels (112, 114, 130) of the device may be provided in a microfluidic system connecting the pulsation dampers (132, 134) and mixer (120), and other components (*e.g.* valves, flow rate sensor, pressure sensor, bubble trapper) according to a working configuration of the device (100). The microfluidic may be formed by additive manufacturing, moulding, milling and the like. Other components may also be formed in the microfluidic system such as the pulsation damper (132, 134) and mixer (120). The microfluid system may be in a multi-well plate format.

The device (100) may be integrated into a micro-fluidic or nano-fluidic system for lab-on-a-chip applications. The micro-fluidic or nano-fluidic system may be formed from glass or silicon.

The device (100) may comprise a control unit, configured to receive signals from one or more of the sensors (*e.g.* flow rate sensors (142), pressure sensors). The control unit may be configured to control flow rate of at least one of the pumps (202, 204), optionally according to a closed feedback loop. The control unit may be configured to control the array of selectable pulsation dampeners (132ac, 134ac), optionally according to a closed feedback loop.

In an optional configuration, the control loop may be used to compensate for the pump pulsations by accurately varying the pump speed (*e.g.* in phase opposition with the pulsation) in order to create a steady state flow, free of pulsation, essentially acting as a pulsation dampener.

The device (100) may be used in any application where accurate inline mixing is desired.

The device (100) may be used for accurate (inline) dilution of a sample. One inlet flow channel in the set (110) may be a sample inlet flow channel (112) configured for conducting a sample flow from a pump (202). One inlet flow channel in the set (110) may be a diluent inlet flow channel (114) configured for conducting a diluent flow from a different pump (204). The mixed and diluted sample flows from mixer (120) outlet. The outlet flow channel (130) may conduct the mixed and diluted sample flow from the mixer (120).

One application for accurate dilution is uninterrupted or periodic monitoring of a growth or liquid culture. A growth or liquid culture may contain cells (*e.g.* micro-organisms, bacterial, yeast, insect cells, mammalian cells, viruses, blood cells, fibroblasts), proteins *(*e.g. immunoglobulins, antibodies), nucleic acid, or any other biological substance. The growth or liquid culture may be in a bioreactor. As cells grow, the density increases, and dilution is necessary in order to fit within a measurement range of a (*e.g.* optical) measurement instrument *(e.g.* spectrophotometer, DHM). Parameters measured *(e.g.* concentration) are corrected by the dilution factor to determine the parameter in the original sample.

Preferably, the device (100) is used for accurate inline dilution of a liquid culture. A liquid culture comprises a supporting liquid medium and at least one population of biological entities. The supporting liquid medium typically comprises salts, nutrients, and a carbon energy source (*e.g.* glucose) and supports maintenance and/or growth of the one or more biological entities.

A biological entity is a natural or engineered cell, an association of natural or engineered cells; a natural or engineered virion; or a part or component of a natural or engineered cell or of a natural or engineered virion or an association of said part or component *(*e.g. part or component being a natural or engineered biological macromolecule). A biological entity is microscopic or sub-microscopic. It is sized so that it may be suspended in a liquid (*e.g.* cells, viruses) or dissolved in a liquid (polypeptide or polynucleic acid). An association of cells may be for instance, a spheroid or organoid, or a partial formation thereof. An association of macromolecules may be for instance, a dimer, trimer or other multimer, or an aggregate.

The liquid culture comprises at least one population of biological entities. The population typically contains multiple copies of the same biological entity (*e.g.* a population contains yeast cells). The liquid culture may comprise several populations of biological entities (*e.g.* a population of yeast cells and a population of proteins expressed by the yeast cells).

A biological entity is preferably a natural or engineered cell. A natural or engineered cell may be, for instance, prokaryotic, eukaryotic, a micro-organism, bacteria, yeast, insect, mammalian, blood, fibroblast or plant. Most preferably, a biological entity is natural or engineered cell, that is yeast, insect, mammalian, blood, fibroblast. The natural or engineered cell may be may be used as a host for production of a natural or engineered virion, or of a natural or engineered biological macromolecule.

A natural or engineered biological macromolecule may be, for instance a polypeptide (*e.g.* protein or protein fragment), or an association of one or more polypeptides (*e.g.* a multimer), or a polynucleic acid (*e.g.* DNA or RNA).

One inlet flow channel in the set (110) may be a liquid culture (a sample) inlet flow channel (112) configured for conducting a liquid culture (sample) flow from a pump (202). One inlet flow channel in the set (110) may be a diluent inlet flow channel (114) configured for conducting a diluent flow from a different pump (204). The mixed and diluted liquid culture (sample) flows from mixer (120) outlet. The outlet flow channel (130) may conduct the mixed and diluted liquid culture (sample) flow from the mixer (120).

The liquid culture may be in a bioreactor or incubator.

The inline dilution of the liquid culture allows monitoring of the liquid culture over time. In particular, it allows monitoring of a population of biological entities in the liquid culture over time. The monitoring comprises inline dilution of a flow of the liquid culture. The monitoring of the liquid culture preferably comprises measurement of one or more parameters of the inline diluted liquid culture at two or more different points in time. A parameter value measured in the diluted liquid culture is corrected by a dilution factor to determine the parameter value (normalised value) in the undiluted liquid culture.

A parameter may be any parameter that is measurable in the inline diluted liquid culture. In particular, the parameter may be any parameter that is measurable of the biological entity (*e.g.* cell) in the population. A parameter may be any parameter that is measurable using a sensor. Preferably, the sensor measures an electrical property (*e.g.* capacitance), an optical property (*e.g.* microscopic image (two dimensional (2D) or three dimensional (3D)), light absorbance, fluorescence)), or spectrometric property (*e.g.* Infrared). At least one parameter may be determined. At least one sensor may be used. At least one property may be determined.

An optical property may be determined using for instance, light microscope, a DHM, or a spectrophotometer. An electrical property may be determined using, for instance, a capacitance probe. An spectrometric property may be determined using, for instance, a spectrometer.

Examples of parameters include biological entity (*e.g.* cell) count, absorbance, capacitance.

In a preferred aspect, one or more parameters is determined from a microscopic optical image (2D or 3D) of the inline diluted liquid culture, in particular, from each biological entity therein. The one or more parameters may be determined from pixel intensities within the image, in particular, of the biological entity (*e.g.* cell) count. Examples of parameters determined from pixel intensities include granularity, geometry features (2D and 3D) such as cell radius, cell circularity, cell height variance. Using microscopy, features of the cell nucleus may also be determined, such as nuclear size. Analysis of pixel intensities of a biological entity that is a cell allow determination of parameters such as cell viability, cell viral infection state, culture bacterial contamination state.

A parameter is measured per unit volume in the diluted liquid culture, and may be used to determine the parameter per unit volume in the liquid culture. In an example, cell count/ml may be measured by DHM in the diluted liquid culture, and used to determine the cell count/ml in the liquid culture. In another example, nuclear size greater than a threshold may be determined per unit volume in the diluted liquid culture, and may be used to determine the parameter per unit volume in the liquid culture. The parameter may be determined as a function of time.

The described device (100) may be used for monitoring multiple liquid cultures. One or more flow switching valves may be provided for selecting the liquid culture that flows into the inlet flow channel (110) of the device (100). Preferably, one pump (202) serves all the multiple liquid cultures. However, it is also foreseen that each liquid culture is provided with its own pump. A dilution factor is determined for each liquid culture. A dilution factor may be determined dynamically determined for each liquid culture.

The monitoring may be uninterrupted meaning that the liquid culture and diluent flow without interruption into the inlet flow channels (112, 114) and are mixed using the device (100) for a duration of interest. One or more parameters may be measured multiple times during the duration of interest, the quantity of times depending on the frequency of measurement during the duration of interest. An example of uninterrupted measurement is during a period of exponential growth of cells in the liquid culture, where changes in one or more parameters of the diluted liquid culture might occur too fast to justify periodic monitoring.

The monitoring may be periodic meaning that the flow of liquid culture and diluent into the inlet flow channels (112, 114) is interrupted by at least one pause during a duration of interest. Liquid flows into the mixer (120) from the set (110) of inlet flow channels (112, 114) for a flow period, and stops flowing for a subsequent pause period. There are preferably at least two flow periods and at least two pause periods in the duration of interest. Usually, for the duration of interest, the pause periods have the same length and the flow periods have the same length. The pause period length and flow period length may be the same or different. An exemplary periodic flow has a sequence 30 s FLOW - 20 mins PAUSE - 30 s FLOW, and further repetitions of consecutive of PAUSE-FLOW states for the duration of interest. One or more parameters is preferably measured during the pause period. It is not excluded, however, that no measurements is made during the pause period. During the flow period, the previous sample is flushed from the mixer. One or more parameters are preferably not measured during the flow period. It is not excluded, however, that measurements are made during the flow period. An example of periodic measurement is during a period of slow growth of cells in the liquid culture, where changes in one or more parameters of the diluted liquid culture occur slowly and not just justify loss of liquid culture to continuous monitoring. The periodic measurement may be automated.

The duration of interest is a duration of time over which measurements of the liquid culture are desirable. An example of a duration of interest is a period of growth of cells in the liquid culture from inoculation to steady state growth. The duration of interest may last, for instance, several days.

The flow rate of the diluent through the diluent inlet flow channel (114) and/or the flow rate of the liquid culture through the liquid culture inlet flow channel (112) may be dynamically adjustable in response to the monitoring of the diluted liquid culture. By dynamically adjustable, it is meant that can be adjusted to respond in real time the monitoring of the diluted liquid culture, for example, using closed-loop feedback. The device (100) may comprise a control unit, configured to receive signals from the monitoring. The control unit may be configured to control flow rate of at least one of the pumps (202, 204) according to the closed-loop feedback. As an example, during a monitoring lasting several days, more diluent may be added towards the final days compared with in earlier days to maintain the diluted liquid culture within range of a sensor or instrument. The dilution factor is also dynamically determined according to the dynamically adjusted liquid culture (first) flow rate and/or diluent (second) flow rate.

A liquid culture is always not amenable to undiluted monitoring because a growth of a culture causes it to exceed the dynamic range of a sensor or instrument, or a parameter being measured in a high-density culture also exceeds the dynamic range of the sensor or instrument. In particular, where each cell is imaged (*e.g.* DHM, microscopy), dilution assists with visualisation of individual cells.

The present device and method allows periodic sampling and accurate monitoring of a liquid culture via the diluted flow over a course of a production cycle. The present device and method allow detection of small changes which would be undetectable using standard dilution methods because of dilution errors. Early detection is desirable because liquid cultures are frequently incubated in large batches for several days or weeks. A small change might be indicative of a contamination or process which needs an intervention. Being able to address a deviation early can prevent loss of a batch.

The present device and method allows periodic sampling and accurate monitoring of a slow-growing culture. Errors associated with standard dilution methods would prevent detection of small changes. The present device and method allows periodic sampling and accurate monitoring of cultures grown under steady-state conditions; with these cultures small changes can be more difficult to detected against a noise background.

As mentioned previously, the inventors have found that the presence of a mixer introduces an unknown delay, that can be different for each inlet between the flow produced by the pump, and the fraction of each inlet flow constituting the mixed output. Factors influencing the delay are numerous and include flow properties of the inlet material and the pump pulsating frequency. If the flow rates in each inlet flow channel (112, 114) are measured with inline flow rate sensors (142, 144), a correction applied to account for the flow rate variation would still yield an erroneous measurement of the mixing ratio. The present device (100) overcomes the problem of the mixer and allows accurate monitoring of the liquid culture.

The device (100) further allows automated monitoring of a liquid culture. Sample of the liquid culture may be withdrawn by the pump, diluted using the device (100), and one or more parameters measured. The monitoring may proceed automatically (*e.g.* without manual steps) for several hours, days or weeks. As mentioned previously, the flow rate of the diluent through the diluent inlet flow channel (114) and/or the flow rate of the liquid culture through the liquid culture inlet flow channel (112) may be dynamically adjustable in response to the monitoring of the diluted liquid culture.

The fact that the device (100) is inline, means that the mixer (120) is flushed and hence cleaned when liquid culture and/or diluent flow into the mixer (120). This compared with standard batch-mode dilutions in which the mixing container must be cleaned and dried with each reading.

### Example

A culture of yeast cells was grown in a cell-culture medium in a bioreactor. From the cell culture, two different batches were prepared at different concentrations (80 million cells/ml (80CML) and 1000 million cells/ml (1000CML). For each batch, cells were drawn off by a peristaltic pump and diluted by a diluent supplied by a separate peristaltic pump using a device as described herein having two inlet flow channels each provided with a flow meter and pulsation dampener downstream. In one set of experiments, the pulsation dampeners were activated, and in another set of experiments, the pulsation dampeners were deactivated. Concentration of cells was measured on both 80CML and 1000CML batches at five different points in time and CV (Coefficient of variation, which is a ratio between the standard deviation and the mean) was computed for the set of 5 points. Concentration measurements were performed using a DHM and a set of automatic cell counting algorithms.

For batch 80CML without dampening CV was 7.9%; with dampening there was a reduction in CV of 27%. For batch 1000CML without dampening CV was 5.0%; with dampening there was a reduction in CV of 56%.

## Claims

1. Method for monitoring a liquid culture, wherein the monitoring comprises inline dilution of a flow of the liquid culture and dynamically determining a dilution factor of the liquid culture, comprising:
- providing a device (100) for inline mixing of two or more liquid flows, wherein each liquid flow is induced by a separate pump (202, 204), the device (100) comprising:
- a set (110) of inlet flow channels (112, 114) each configured for conducting a liquid flow from the separate pump (202, 204),
- a mixer (120) into which liquid from the set (110) of inlet flow channels (112, 114) enters and is mixed,
wherein passage of liquid flow through each inlet flow channel (112, 114) effects a reduction in periodic variability in liquid flow rate.
wherein
- each inlet flow channel (112, 114) of the set (110) is disposed with a pulsation dampener (132, 134), configured to effect the reduction in periodic variability in liquid flow rate.
- at least one inlet flow channel (110) of the set (110) of inlet flow channels (112, 114) is a liquid culture inlet flow channel (112) and conducts the liquid culture flow from the separate pump (202); and
- at least one inlet flow channel (110) of the set (110) is a diluent inlet flow channel (114) and conducts a diluent flow from the separate pump (204);
- - diluting the liquid culture flow with the diluent flow using the device (100), thereby obtaining an inline diluted liquid culture;
- measuring a first flow rate in the liquid culture inlet flow channel (112) downstream of the pulsation dampener (132);
- measuring a second flow rate in the diluent inlet flow channel (114) downstream of the pulsation dampener (134);
- wherein:
- the dilution factor is a ratio between the sum of the first flow rate and the second flow rate, and the first flow rate, and is dynamically determined responsive to changes in the first flow rate and/or second flow rate;
- the monitoring of the liquid culture is determined from the diluted liquid culture and the dilution factor.

2. The method according to claim 1, wherein the device further comprises a flow rate sensor (142, 144) provided for each inlet flow channel (112, 114), configured to measure the flow of liquid in the respective inlet flow channel (112, 114).

3. The method according to claim 1 or 2, wherein the device is provided with multiple selectable pulsation dampeners (132a to 132c, 134a to 134c), arranged such that each inlet flow channel (112, 114) is provided with a set (132ac or 134ac) of at least two selectable pulsation dampeners (132a-c; 134a-c) connected in parallel.

4. The method according to claim 3, wherein each selectable pulsation dampener within a set (132ac or 134ac) has a different performance.

5. The method according to claim 3 or 4, wherein the device is provided with a controller configured for automatic selection of a selectable pulsation dampeners (132a to 132c, 134a to 134c) responsive to flow rate through the inlet flow channels (112, 114).

6. The method according any one of claims 1 to 5, wherein the mixer (120) is passive, wherein the passive mixer does not contain a powered mixing element.

7. The method according to any claims 1 to 6, wherein the device is provided with an active or passive bubble trapper.

8. The method according to any of claims 1 to 7, wherein the device is provided in a microfluidic system.

9. The method according to any of claims 1 to 8, wherein the monitoring comprises measurement of one or more parameters using digital holographic microscopy.

10. The method according to any of claims 1 to 9, wherein the monitoring is uninterrupted or periodic.

11. The method according to any of claims 1 to 10, wherein the flow rate of the diluent through the diluent inlet flow channel (114) and/or the flow rate of the liquid culture through the liquid culture inlet flow channel (112) is dynamically adjustable in response to the monitoring of the diluted liquid culture.

12. The method according to any one of claims 1 to 11, wherein the liquid culture comprises one or more of:
- natural or engineered cells an association of natural or engineered cells (wherein the cell is a cell such as prokaryotic, eukaryotic, micro-organism, bacteria, yeast, insect, mammalian, blood, fibroblast or plant);
- natural or engineered virus;
- part or component of a natural or engineered cell or of a natural or engineered virion or an association of said part or component (wherein the part or component is a natural or engineered biological macromolecule, such as an immunoglobulins, antibody, nucleic acid).

## Patentansprüche

1. Verfahren zum Überwachen einer Flüssigkultur, wobei das Überwachen Inline-Verdünnung eines Stroms der Flüssigkultur und dynamisches Bestimmen eines Verdünnungsfaktors der Flüssigkultur umfasst, umfassend:
- Bereitstellen einer Vorrichtung (100) zum Inline-Mischen von zwei oder mehr Flüssigkeitsströmen, wobei jeder Flüssigkeitsstrom durch eine separate Pumpe (202, 204) induziert ist, wobei die Vorrichtung (100) Folgendes umfasst:
- ein Satz (110) von Einlass-Strömungskanälen (112, 114), die jeweils so ausgelegt sind, dass ein Flüssigkeitsstrom aus der separaten Pumpe (202, 204) geleitet wird,
- einen Mischer (120), in den Flüssigkeit aus dem Satz (110) von Einlass-Strömungskanälen (112, 114) eintritt und gemischt wird,
wobei Durchlaufen des Flüssigkeitsstroms durch jeden Einlass-Strömungskanal (112, 114) eine Reduzierung von periodischer Variabilität der Flüssigkeitsfließgeschwindigkeit bewirkt.
wobei
- jeder Einlass-Strömungskanal (112, 114) des Satzes (110) mit einem Pulsationsdämpfer (132, 134) versehen ist, der so ausgelegt ist, dass die Reduzierung von periodischer Variabilität der Flüssigkeitsfließgeschwindigkeit bewirkt wird.
- es sich bei mindestens einem Einlass-Strömungskanal (110) des Satzes (110) von Einlass-Strömungskanälen (112, 114) um einen Flüssigkultur-Einlass-Strömungskanal (112) handelt und dieser den Flüssigkulturstrom aus der separaten Pumpe (202) leitet; und
- es sich bei mindestens einem Einlass-Strömungskanal (110) des Satzes (110) um einen Verdünnungsmittel-Einlass-Strömungskanal (114) handelt und dieser einen Verdünnungsmittelstrom aus der separaten Pumpe (204) leitet;
- Verdünnen des Flüssigkulturstroms mit dem Verdünnungsmittelstrom unter Verwendung der Vorrichtung (100), wodurch eine inline-verdünnte Flüssigkultur erhalten wird;
- Messen einer ersten Fließgeschwindigkeit im Flüssigkultur-Einlass-Strömungskanal (112) stromabwärts des Pulsationsdämpfers (132);
- Messen einer zweiten Fließgeschwindigkeit im Verdünnungsmittel-Einlass-Strömungskanal (114) stromabwärts des Pulsationsdämpfers (134);
- wobei:
- der Verdünnungsfaktor ein Verhältnis von der Summe der ersten Fließgeschwindigkeit und der zweiten Fließgeschwindigkeit zur ersten Fließgeschwindigkeit darstellt und dynamisch auf Änderungen der ersten Fließgeschwindigkeit und/oder zweiten Fließgeschwindigkeit ansprechend, bestimmt wird;
- das Überwachen der Flüssigkultur aus der verdünnten Flüssigkultur und dem Verdünnungsfaktor bestimmt wird.

2. Verfahren nach Anspruch 1, wobei die Vorrichtung ferner einen Fließgeschwindigkeitssensor (142, 144) umfasst, der für jeden Einlass-Strömungskanal (112, 114) bereitgestellt ist und so ausgelegt ist, dass der Fluss von Flüssigkeit in dem jeweiligen Einlass-Strömungskanal (112, 114) gemessen wird.

3. Verfahren nach Anspruch 1 oder 2, wobei die Vorrichtung mit mehreren auswählbaren Pulsationsdämpfern (132a bis 132c, 134a bis 134c) ausgestattet ist, die so angeordnet sind, dass jeder Einlass-Strömungskanal (112, 114) mit einem Satz (132ac oder 134ac) von mindestens zwei auswählbaren Pulsationsdämpfern (132a-c; 134a-c), die parallel verbunden sind, ausgestattet ist.

4. Verfahren nach Anspruch 3, wobei jeder auswählbare Pulsationsdämpfer innerhalb eines Satzes (132ac oder 134ac) eine unterschiedliche Leistung aufweist.

5. Verfahren nach Anspruch 3 oder 4, wobei die Vorrichtung mit einem Controller ausgestattet ist, die auf automatische Auswahl eines auf die Fließgeschwindigkeit durch den Inlet-Strömungskanal (112, 114) ansprechenden auswählbaren Pulsationsdämpfers (132a bis 132c, 134a bis 134c) ausgelegt ist.

6. Verfahren nach einem der Ansprüche 1 bis 5, wobei der Mischer (120) passiv ist, wobei der passive Mischer kein angetriebenes Mischelement enthält.

7. Verfahren nach einem der Ansprüche 1 bis 6, wobei die Vorrichtung mit einem aktiven oder passiven Blasenfänger ausgestattet ist.

8. Verfahren nach einem der Ansprüche 1 bis 7, wobei die Vorrichtung in einem mikrofluidischen System bereitgestellt ist.

9. Verfahren nach einem der Ansprüche 1 bis 8, wobei das Überwachen Messung ein oder mehrerer Parameter unter Verwendung von digital-holographischer Mikroskopie umfasst.

10. Verfahren nach einem der Ansprüche 1 bis 9, wobei das Überwachen ununterbrochen oder periodisch erfolgt.

11. Verfahren nach einem der Ansprüche 1 bis 10, wobei die Fließgeschwindigkeit des Verdünnungsmittels durch den Verdünnungsmittel-Einlass-Strömungskanal (114) und/oder die Fließgeschwindigkeit der Flüssigkultur durch den Flüssigkultur-Einlass-Strömungskanal (112) als Reaktion auf das Überwachen der verdünnten Flüssigkultur dynamisch anpassbar ist.

12. Verfahren nach einem der Ansprüche 1 bis 11, wobei die Flüssigkultur ein oder mehrere der Folgenden umfasst:
- natürliche oder konstruierte Zellen, eine Sammlung von natürlicher oder konstruierter Zellen (wobei es sich bei der Zelle um beispielsweise eine prokaryotische, eukaryotische, Mikroorganisme, bakterielle, Hefe-, Insekten-, Säugetier-, Blut-, Fibroblasten oder Pflanzenzelle handelt);
- natürliches oder konstruiertes Virus;
- Teil oder Komponente einer natürlichen oder konstruierten Zelle oder eines natürlichen oder konstruierten Virions oder eine Sammlung des Teils oder der Komponente (wobei der Teil oder die Komponente ein natürliches oder konstruiertes biologisches Makromolekül ist, wie ein Immunglobulin, Antikörper, eine Nukleinsäure).

## Revendications

1. Procédé de surveillance d'une culture liquide, dans lequel la surveillance comprend la dilution en ligne d'un flux de la culture liquide et la détermination dynamique d'un facteur de dilution de la culture liquide, comprenant :
- la fourniture d'un dispositif (100) pour le mélange en ligne de deux ou plusieurs flux liquides, chaque flux liquide étant induit par une pompe distincte (202, 204), le dispositif (100) comprenant :
- un ensemble (110) de canaux de flux d'entrée (112, 114) configurés chacun pour conduire un flux liquide provenant de la pompe distincte (202, 204),
- un mélangeur (120) dans lequel un liquide provenant de l'ensemble (110) de canaux de flux d'entrée (112, 114) pénètre et est mélangé,
le passage du flux liquide à travers chaque canal de flux d'entrée (112, 114) effectuant une réduction de la variabilité périodique du débit de liquide.
dans lequel
- chaque canal de flux d'entrée (112, 114) de l'ensemble (110) est doté d'un amortisseur de pulsations (132, 134) configuré pour réduire la variabilité périodique du débit de liquide ;
- au moins un canal de flux d'entrée (110) de l'ensemble (110) de canaux de flux d'entrée (112, 114) est un canal de flux d'entrée de culture liquide (112) et conduit le flux de culture liquide provenant de la pompe distincte (202) ; et
- au moins un canal de flux d'entrée (110) de l'ensemble (110) est un canal de flux d'entrée de diluant (114) et conduit un flux de diluant provenant de la pompe distincte (204) ;
- le flux de culture liquide est dilué avec le flux de diluant en utilisant le dispositif (100), ce qui permet d'obtenir une culture liquide diluée en ligne ;
- la mesure d'un premier débit dans le canal de flux d'entrée de culture liquide (112) en aval de l'amortisseur de pulsations (132) ;
- la mesure d'un second débit dans le canal de flux d'entrée de diluant (114) en aval de l'amortisseur de pulsations (134) ;
- dans lequel :
- le facteur de dilution est un rapport entre la somme du premier débit et du deuxième débit, et le premier débit, et est déterminé dynamiquement en réponse à des changements du premier débit et/ou du deuxième débit ;
- la surveillance de la culture liquide est déterminée provenant de la culture liquide diluée et du facteur de dilution.

2. Procédé selon la revendication 1, dans lequel le dispositif comprend en outre un capteur de débit (142, 144) fourni pour chaque canal de flux d'entrée (112, 114), configuré pour mesurer le flux de liquide dans le canal de flux d'entrée respectif (112, 114).

3. Procédé selon la revendication 1 ou 2, dans lequel le dispositif est muni de multiples amortisseurs de pulsations sélectionnables (132a à 132c, 134a à 134c), agencés de telle sorte que chaque canal de flux d'entrée (112, 114) est muni d'un ensemble (132ac ou 134ac) d'au moins deux amortisseurs de pulsations sélectionnables (132a-c ; 134a-c) connectés en parallèle.

4. Procédé selon la revendication 3, dans lequel chaque amortisseur de pulsations sélectionnable dans un ensemble (132ac ou 134ac) a une performance différente.

5. Procédé selon la revendication 3 ou 4, dans lequel le dispositif est muni d'un dispositif de contrôle configuré pour la sélection automatique d'un amortisseur de pulsations sélectionnable (132a à 132c, 134a à 134c) en réponse au débit à travers les canaux de flux d'entrée (112, 114).

6. Procédé selon l'une quelconque des revendications 1 à 5, dans lequel le mélangeur (120) est passif, dans lequel le mélangeur passif ne contient pas un élément mélangeur alimenté.

7. Procédé selon l'une quelconque des revendications 1 à 6, dans lequel le dispositif est muni d'un piège à bulles actif ou passif.

8. Procédé selon l'une quelconque des revendications 1 à 7, dans lequel le dispositif est fourni dans un système microfluidique.

9. Procédé selon l'une quelconque des revendications 1 à 8, dans lequel la surveillance comprend la mesure d'un ou plusieurs paramètres en utilisant la microscopie holographique numérique.

10. Procédé selon l'une quelconque des revendications 1 à 9, dans lequel la surveillance est ininterrompue ou périodique.

11. Procédé selon l'une quelconque des revendications 1 à 10, dans lequel le débit du diluant à travers le canal de flux d'entrée de diluant (114) et/ou le débit de la culture liquide à travers le canal de flux d'entrée de culture liquide (112) est réglable dynamiquement en réponse à la surveillance de la culture liquide diluée.

12. Procédé selon l'une quelconque des revendications 1 à 11, dans lequel la culture liquide comprend un(e) ou plusieurs parmi :
- des cellules naturelles ou modifiées, une association de cellules naturelles ou modifiées (dans laquelle la cellule est une cellule telle que procaryote, eucaryote, micro-organisme, bactérie, levure, insecte, mammifère, sang, fibroblaste ou végétal) ;
- un virus naturel ou modifié ;
- une partie ou un composant d'une cellule naturelle ou modifiée ou d'un virion naturel ou modifié ou une association de ladite partie ou dudit composant (la partie ou le composant étant une macromolécule biologique naturelle ou modifiée, telle qu'une immunoglobuline, un anticorps, un acide nucléique).
